# EUROPEAN PATENT APPLICATION

(11) **EP 4 696 774 A1**
(43) Date of publication of application: **18.02.2026**
(21) Application number: 24815872.7
(22) Date of filing: 30.05.2024
(51) Int. Cl.: C12N 9/10, C12N 15/70, C12P 13/12

(54) **O-PHOSPHOSERINE SULFHYDRYLASE VARIANT AND METHOD FOR PRODUCING CYSTEINE USING SAME**

(30) Priority: 01.06.2023 KR 20230070989
(71) Applicant: CJ Cheiljedang Corporation, Seoul 04560 (KR)
(72) Inventor: SIM, Hee-jin, Seoul 04560 (KR); RHO, Jin Ah, Seoul 04560 (KR); CHOI, Jin-Geun, Seoul 04560 (KR); KIM, Moonjung, Seoul 04560 (KR)
(74) Representative: Meissner Bolte Partnerschaft mbB
(86) International application number: PCT/KR2024/007386
(87) International publication number: WO 2024/248498

(57) **Abstract**

The present disclosure relates to an O-phosphoserine sulfhydrylase variant and a method for producing cysteine using the same.

## Description

### [Technical Field]

The present disclosure relates to an O-phosphoserine sulfhydrylase variant and a method for producing cysteine using the same.

### [Background Art]

L-cysteine, an amino acid playing an important role in sulfur metabolism in all living organisms, is used not only in the synthesis of biological proteins such as hair keratin, *etc.,* glutathione, biotin, methionine, and other sulfur-containing metabolites, but also as a precursor for biosynthesis of' coenzyme A.

Methods of producing L-cysteine using microorganisms known in the art include: 1) a method of biologically converting D,L-2-aminothiazoline-4-carboxylic acid (D,L-ATC) into L-cysteine using microorganisms, 2) a method of producing L-cysteine by direct fermentation using *E*. *coli* (EP0885962B), and 3) a method of producing O-phosphoserine (hereinafter "OPS") by fermentation using microorganisms, and then converting O-phosphoserine into L-cysteine by reacting O-phosphoserine with a sulfide under the catalytic action of O-phosphoserine sulfhydrylase (hereinafter "OPSS") (US 8557549 B2), *etc.*

In particular, in order to produce cysteine by the method 3) at high yield, OPS, the precursor, should be produced in excessive amounts.

### [Disclosure]

### [Technical Problem]

The present inventors confirmed that cysteine can be produced at high yield when using the O-phosphoserine sulfhydrylase variant of the present disclosure in a cysteine-producing microorganism, as compared to using a microorganism having an existing wild-type O-phosphoserine sulfhydrylase variant, and thus completed the present disclosure.

### [Technical Solution]

It is one object of the present disclosure to provide an O-phosphoserine sulfhydrylase variant, in which 0 to 7 amino acid residues from the C-terminus are deleted in the amino acid sequence of SEQ ID NO: 1, and the amino acid corresponding to the position 77 is substituted with alanine.

It is another object of the present disclosure to provide a polynucleotide encoding the O-phosphoserine sulfhydrylase variant of the present disclosure.

It is still another object of the present disclosure to provide a microorganism comprising the O-phosphoserine sulfhydrylase variant of the present disclosure; or a polynucleotide encoding the same.

It is yet another object of the present disclosure to provide a method for producing cysteine or a derivative thereof, comprising reacting a mixture of O-phosphoserine and a sulfide; with the O-phosphoserine sulfhydrylase variant of the present disclosure.

### [Advantageous Effects]

Cysteine can be produced at high yield when culturing a cysteine-producing microorganism using the O-phosphoserine sulfhydrylase variant of the present disclosure, as compared to using a microorganism having an existing wild-type O-phosphoserine sulfhydrylase variant.

### [Brief Description of Drawing]

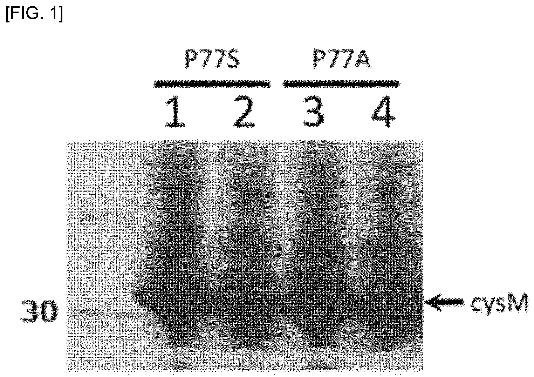

FIG. 1 shows K12/pUC_Ppro-cysM(P77S) (Msm-T-HA2) and K12/pUC_Ppro-cysM(P77A), which are CysM variants, confirmed through SDS-PAGE.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification, and their citations are indicated. The content of the cited papers and patent documents is incorporated herein by reference in their entirety and the level of technical field to which the present disclosure belongs and the contents of the present disclosure will be described more clearly.

One aspect of the present disclosure provides an O-phosphoserine sulfhydrylase variant, in which 0 to 7 amino acid residues from the C-terminus are deleted in the amino acid sequence of SEQ ID NO: 1, and the amino acid corresponding to the position 77 is substituted with alanine.

As used herein, the term "O-phosphoserine sulfhydrylase (OPSS)" refers to an enzyme that catalyzes a reaction by which OPS is converted into cysteine by providing a thiol group (SH group) to OPS. The enzyme may have been first found in *Aeropyrum pernix, Mycobacterium tuberculosis, Mycobacterium smegmatis*, and *Trichomonas vaginalis* (Mino K and Ishikawa K, FEBS Letters, 551: 133-138, 2003; Burns K E et al., J. Am. Chem. Soc., 127: 11602-11603, 2005).

The O-phosphoserine sulfhydrylase of the present disclosure may include any polypeptide having the O-phosphoserine sulfhydrylase activity or any O-phosphoserine sulfhydrylase. The polypeptide having the O-phosphoserine sulfhydrylase activity or the O-phosphoserine sulfhydrylases may include any polypeptide having the O-phosphoserine sulfhydrylase activity or an activity of converting O-phosphoserine as a substrate into cysteine. In one example, the polypeptide having the O-phosphoserine sulfhydrylase activity may be any polypeptide having the O-phosphoserine sulfhydrylase activity derived from microorganisms or the activity of converting O-phosphoserine as a substrate into cysteine. Specifically, the polypeptide having the O-phosphoserine sulfhydrylase activity of the present disclosure may be derived from a prokaryotic microorganism or a eukaryotic microorganism, and more specifically derived from a microorganism of the genus *Mycobacterium*, *etc*., but is not limited thereto. In another example, the polypeptide having the O-phosphoserine sulfhydrylase activity of the present disclosure may be a cysteine synthase (CysM) derived from a microorganism of the genus *Mycobacterium.* The amino acid sequence of CysM can be obtained from GenBank of NCBI, a known database, *etc.,* and for example, it may be WP_003896302.1 derived from a microorganism of the genus *Mycobacterium,* but it is apparent that any protein having the O-phosphoserine sulfhydrylase activity or the activity of converting OPS as a substrate into cysteine from various origins may be included.

Additionally, the O-phosphoserine sulfhydrylase may include not only wild-type O-phosphoserine sulfhydrylase proteins, but also variant proteins, in which a part of the sequence is deleted, substituted, or added in the polynucleotide sequence encoding the O-phosphoserine sulfhydrylase, which show activity that is equal to or higher than the biological activity of the wild-type O-phosphoserine sulfhydrylase proteins, and may also include all O-phosphoserine sulfhydrylase proteins disclosed in EP 2444481 A1 and US 2012-0190080 A, and their variant proteins.

The "O-phosphoserine sulfhydrylase" may be referred to as "OPSS", "cysteine synthase", "CysM", *etc.*

As used herein, the term "O-phosphoserine sulfhydrylase (OPSS) variant" may refer to a variant, in which 0 to 7 amino acid residues from the C-terminus are deleted in the amino acid sequence of SEQ ID NO: 1, and the amino acid corresponding to the position 77 is substituted with alanine in any polypeptide having the O-phosphoserine sulfhydrylase activity or O-phosphoserine sulfhydrylase.

The O-phosphoserine sulfhydrylase variant may be referred to as a "modified O-phosphoserine sulfhydrylase", "OPSS variant", "modified OPSS", "CysM variant", "modified CysM", *etc*.

The protein targeted for the introduction of mutation of the present disclosure may be a protein having the O-phosphoserine sulfhydrylase activity or the activity of converting O-phosphoserine as a substrate into cysteine. Specifically, the protein may have the O-phosphoserine sulfhydrylase activity comprising the amino acid sequence of SEQ ID NO: 1 or the activity of converting O-phosphoserine as a substrate into cysteine, but is not limited thereto. The protein does not exclude an addition of a meaningless sequence upstream or downstream of the amino acid sequence of SEQ ID NO: 1, a naturally occurring mutation, or a silent mutation therein, and any protein may fall within the scope of the protein targeted for the introduction of mutation of the present disclosure as long as the protein has activity identical or corresponding to the activity of the protein comprising the amino sequence of SEQ ID NO: 1. For example, the protein targeted for the introduction of mutation of the present disclosure may be a protein consisting of the amino acid sequence of SEQ ID NO: 1 or an amino acid sequence having a homology or identity of 80%, 85%, 90%, 95%, 96%, 97%, 98%, or 99% or higher thereto. Additionally, it is apparent that any protein having an amino acid sequence, in which part of the sequence is deleted, modified, substituted, or added, may also fall within the scope of the protein targeted for mutation of the present disclosure as long as the amino acid sequence has such a homology or identity, and exhibits an efficacy corresponding to the protein. Example of the protein targeted for mutation of the present disclosure, i.e., a parent sequence, may include the amino acid sequence of SEQ ID NO: 1 or SEQ ID NO: 13.

The O-phosphoserine sulfhydrylase variant of the present disclosure may be one in which 0 to 7 amino acid residues from the C-terminus are deleted in the amino acid sequence of SEQ ID NO: 1, and the amino acid corresponding to the position 77 from the N-terminus is substituted with an amino acid other than the amino acid before substitution in the amino acid sequence of SEQ ID NO: 1. Specifically, the O-phosphoserine sulfhydrylase variant of the present disclosure may be one in which 3 to 7 amino acid residues from the C-terminus are deleted in the amino acid sequence of SEQ ID NO: 1, and the amino acid corresponding to the position 77 from the N-terminus is substituted with an amino acid other than the amino acid before substitution in the amino acid sequence of SEQ ID NO: 1. More specifically, the O-phosphoserine sulfhydrylase variant of the present disclosure may be one in which 5 amino acid residues from the C-terminus are deleted in the amino acid sequence of SEQ ID NO: 1, and the amino acid corresponding to the position 77 from the N-terminus is substituted with an amino acid other than the amino acid before substitution in the amino acid sequence of SEQ ID NO: 1. The amino acid corresponding to the position 77 in the amino acid sequence of SEQ ID NO: 1 of the O-phosphoserine sulfhydrylase protein targeted for the introduction of mutation in the present disclosure, before substitution, may be proline (P).

In one example, the O-phosphoserine sulfhydrylase variant may be one in which the amino acid corresponding to the position 77 of SEQ ID NO: 1 is substituted with an amino acid other than proline. In another example, the O-phosphoserine sulfhydrylase variant may be on in which the amino acid corresponding to the position 77 of SEQ ID NO: 1 is substituted with an amino acid selected from the group consisting of alanine, tyrosine, arginine, lysine, aspartic acid, asparagine, valine, leucine, isoleucine, methionine, phenylalanine, tryptophan, threonine, cysteine, histidine, glycine, glutamic acid and glutamine, and in one example, alanine. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan and tyrosine; and hydrophobic amino acids include alanine, valine, isoleucine, leucine, methionine, phenylalanine, tyrosine, and tryptophan.

In addition, amino acids can be classified into amino acids with electrically charged side chains and amino acids with uncharged side chains. Amino acids with electrically charged side chains include aspartic acid, glutamic acid, lysine, arginine, and histidine; and amino acids with uncharged side chains can be further classified into nonpolar amino acids or polar amino acids. Nonpolar amino acids include glycine, alanine, valine, leucine, isoleucine, methionine, phenylalanine, tryptophan, and proline; and polar amino acids include serine, threonine, cysteine, tyrosine, asparagine, and glutamine. Typically, conservative substitutions may have little or no effect on the activity of a protein or polypeptide.

In one embodiment, the O-phosphoserine sulfhydrylase variant of the present disclosure may have, include, or consist of the amino acid sequence represented by SEQ ID NO: 3, or may essentially consist of the amino acid sequence above. The O-phosphoserine sulfhydrylase variant of the present disclosure may include an amino acid sequence having a homology or identity of at least 70%, 75%, 80%, 85%, 90%, 95%, 96%, 97%, 98%, 99%, 99.1%, 99.2%, 99.3%, 99.4%, 99.5%, 99.6%, 99.7%, 99.8%, or 99.9% or higher to the amino acid sequence of SEQ ID NO: 3. Additionally, it is apparent that any O-phosphoserine sulfhydrylase variant having an amino acid sequence, in which part of the sequence is deleted, modified, substituted, conservatively substituted or added, may also fall within the scope of the present disclosure as long as the amino acid sequence has such a homology or identity, and exhibits an efficacy corresponding to the O-phosphoserine sulfhydrylase variant of the present disclosure.

For example, it may be sequence additions or deletions that do not alter the function of the O-phosphoserine sulfhydrylase variant of the present disclosure, naturally occurring mutations, silent mutations therein, or conservative substitutions at the N-terminus, C-terminus and/or within the amino acid sequences.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of a protein or polypeptide.

The O-phosphoserine sulfhydrylase variant of the present disclosure may be one in which the amino acid corresponding to the positions selected from the group consisting of 7, 55, 218, and a combination thereof from the N-terminus in the amino acid sequence of SEQ ID NO: 1 is further substituted with an amino acid other than the amino acid before substitution.

The amino acid corresponding to the 7^{th} position of SEQ ID NO: 1 before substitution may be leucine (L), the amino acid corresponding to the 55^{th} position before substitution may be alanine (A), and the amino acid corresponding to the 218^{th} position before substitution may be alanine (A), in the amino acid sequence of the O-phosphoserine sulfhydrylase protein targeted for the introduction of mutation in the present disclosure, before substitution.

The O-phosphoserine sulfhydrylase variant of the present disclosure may be, in one example, one in which the amino acid corresponding to the 7^{th} position of SEQ ID NO: 1 may be substituted with an amino acid other than leucine. In another example, the O-phosphoserine sulfhydrylase variant may be one in which the amino acid corresponding to the 7^{th} position of SEQ ID NO: 1 may be substituted with an amino acid selected from the group consisting of proline, tyrosine, glycine, alanine, aspartic acid, glutamic acid, histidine, valine, lysine, arginine, isoleucine, methionine, phenylalanine, tryptophan, serine, threonine, cysteine, asparagine, and glutamine, and in one example, proline.

The O-phosphoserine sulfhydrylase variant of the present disclosure may be, in one example, one in which the amino acid corresponding to the 55^{th} position of SEQ ID NO: 1 may be substituted with an amino acid other than alanine. In another example, the O-phosphoserine sulfhydrylase variant may be one in which the amino acid corresponding to the 55^{th} position of SEQ ID NO: 1 may be substituted with an amino acid selected from the group consisting of valine, tyrosine, glycine, leucine, aspartic acid, glutamic acid, histidine, lysine, arginine, isoleucine, methionine, proline, phenylalanine, tryptophan, serine, threonine, cysteine, asparagine, and glutamine, and in one example, valine.

The O-phosphoserine sulfhydrylase variant of the present disclosure may be, in one example, one in which the amino acid corresponding to the 218^{th} position of SEQ ID NO: 1 may be substituted with an amino acid other than alanine. In another example, the O-phosphoserine sulfhydrylase variant may be one in which the amino acid corresponding to the 218^{th} position of SEQ ID NO: 1 may be substituted with an amino acid selected from the group consisting of glycine, tyrosine, leucine, aspartic acid, glutamic acid, histidine, valine, lysine, arginine, isoleucine, methionine, proline, phenylalanine, tryptophan, serine, threonine, cysteine, asparagine and glutamine, and in one example, glycine.

In one embodiment, the O-phosphoserine sulfhydrylase variant of the present disclosure may be one in which 0 to 7, e.g., 3 to 7 amino acid residues from the C-terminus in the amino acid sequence of SEQ ID NO: 1 are deleted, and which further includes one or more, two or more, or all three amino acid substitutions at position corresponding to 7^{th}, 5^{th}, or 218^{th} from the N-terminus in the amino acid sequence of SEQ ID NO: 1, while the amino acid substitution at position corresponding to 77^{th} from the N-terminus in the amino acid sequence of SEQ ID NO: 1 is fixed.

In another embodiment, the O-phosphoserine sulfhydrylase variant of the present disclosure may have, include, or consist of any one or more amino acid sequence selected from the group consisting of SEQ ID NOS: 5, 7, and 9, or may essentially consist of the amino acid sequence above. The O-phosphoserine sulfhydrylase variant of the present disclosure may include an amino acid sequence having a homology or identity of at least 70%, 75%, 80%, 85%, 90%, 95%, 96%, 97%, 98%, 99%, 99.1%, 99.2%, 99.3%, 99.4%, 99.5%, 99.6%, 99.7%, 99.8% or 99.9% or higher to the amino acid sequence of SEQ ID NO: 5, SEQ ID NO: 7, or SEQ ID NO: 9. Additionally, it is apparent that any O-phosphoserine sulfhydrylase variant having an amino acid sequence, in which part of the sequence is deleted, modified, substituted, conservatively substituted or added, may also fall within the scope of the present disclosure as long as the amino acid sequence has such a homology or identity, and exhibits an efficacy corresponding to the O-phosphoserine sulfhydrylase variant of the present disclosure.

As used herein, the term "modified protein" or "variant" refers to a polypeptide having one or more amino acids different from the amino acid sequence before mutation of the variant by conservative substitutions and/or modifications such that the functions and properties of the polypeptide are retained. Such variants may generally be identified by modifying one or more of the amino acid sequences of the polypeptide and evaluating the properties of the modified polypeptide. That is, the ability of the variants may be enhanced, unchanged or reduced relative to a polypeptide before mutation. Further, some variants may include those in which one or more regions, such as an N-terminal leader sequence or transmembrane domain, have been removed. Moreover, other variants may include those in which a region has been removed from the N- and/or C-terminal of a mature protein. The term "modified protein" may be used interchangeably with terms such as modification, modified polypeptide, modified protein, mutant, mutein, divergent, *etc.* without limitation, as long as the terms are used to indicate mutation.

For the purpose of the present disclosure, the variant of the present disclosure may be a polypeptide, in which 0 to 7 amino acid residues from the C-terminus in the amino acid sequence of SEQ ID NO: 1 are deleted, and the amino acid corresponding to the position 77 from the N-terminus in the amino acid sequence of SEQ ID NO: 1 is substituted with another amino acid. Specifically, the variant of the present disclosure may be a polypeptide, in which 5 amino acid residues from the C-terminus in the amino acid sequence of SEQ ID NO: 1 are deleted, and the amino acid corresponding to the position 77 from the N-terminus in the amino acid sequence of SEQ ID NO: 1 is substituted with an amino acid other than the amino acid before substitution. In one example, the variant of the present disclosure may be one in which the amino acid corresponding to the position 77 in the amino acid sequence of SEQ ID NO: 1 is substituted with an amino acid selected from the group consisting of alanine, tyrosine, arginine, lysine, aspartic acid, asparagine, valine, leucine, isoleucine, methionine, phenylalanine, tryptophan, threonine, cysteine, histidine, glycine, glutamic acid and glutamine, and in one example, alanine. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan and tyrosine; and hydrophobic amino acids include alanine, valine, isoleucine, leucine, methionine, phenylalanine, tyrosine, and tryptophan.

In addition, amino acids can be classified into amino acids with electrically charged side chains and amino acids with uncharged side chains. Amino acids with electrically charged side chains include aspartic acid, glutamic acid, lysine, arginine, and histidine; and amino acids with uncharged side chains can be further classified into nonpolar amino acids or polar amino acids. Nonpolar amino acids include glycine, alanine, valine, leucine, isoleucine, methionine, phenylalanine, tryptophan, and proline; and polar amino acids include serine, threonine, cysteine, tyrosine, asparagine, and glutamine. Typically, conservative substitutions may have little or no effect on the activity of a protein or polypeptide.

In another example, the variant of the present disclosure may be a polypeptide comprising the amino acid sequence represented by SEQ ID NO: 3, but is not limited thereto.

The variant of the present disclosure may be a polypeptide, in which 5 amino acid residues from the C-terminus in the amino acid sequence of SEQ ID NO: 1 are deleted, and the amino acid corresponding to the position 77 from the N-terminus in the amino acid sequence of SEQ ID NO: 1 is substituted with an amino acid other than the amino acid before substitution, and may be additionally one in which the amino acid corresponding to the position selected from the group consisting of 7, 55, 218, and a combination thereof from the N-terminus in the amino acid sequence of SEQ ID NO: 1 is substituted with an amino acid other than the amino acid before substitution. In one example, the variant of the present disclosure may be one in which the amino acid corresponding to the 7^{th} position in the amino acid sequence of SEQ ID NO: 1 may be substituted with an amino acid selected from the group consisting of proline, tyrosine, glycine, alanine, aspartic acid, glutamic acid, histidine, valine, lysine, arginine, isoleucine, methionine, phenylalanine, tryptophan, serine, threonine, cysteine, asparagine, and glutamine, and in one example, proline. The variant of the present disclosure may be one in which the amino acid corresponding to the 55^{th} position in the amino acid sequence of SEQ ID NO: 1 may be substituted with an amino acid selected from the group consisting of valine, tyrosine, glycine, leucine, aspartic acid, glutamic acid, histidine, lysine, arginine, isoleucine, methionine, proline, phenylalanine, tryptophan, serine, threonine, cysteine, asparagine, and glutamine, and in one example, valine. Additionally, the variant of the present disclosure may be one in which the amino acid corresponding to the 218^{th} position in the amino acid sequence of SEQ ID NO: 1 may be substituted with an amino acid selected from the group consisting of glycine, tyrosine, leucine, aspartic acid, glutamic acid, histidine, valine, lysine, arginine, isoleucine, methionine, proline, phenylalanine, tryptophan, serine, threonine, cysteine, asparagine and glutamine, and in one example, glycine. In another example, the variant of the present disclosure may be a polypeptide comprising the amino acid sequence represented by SEQ ID NO: 5, SEQ ID NO: 7, or SEQ ID NO: 9, but is not limited thereto. The variant may be one which includes the above-described modifications, and in which the enzymatic activity of O-phosphoserine sulfhydrylase is improved as compared to a polypeptide in which the amino acid corresponding to the 77^{th} position from the N-terminus in the amino acid sequence of SEQ ID NO: 1 is proline.

Additionally, the variant may also include deletion or addition of amino acids that have minimal influence on the properties and secondary structure of a polypeptide. For example, the polypeptide may be conjugated with a signal (or leader) sequence at the N-terminal involved in the translocation of proteins co-translationally or post-translationally. Further, the polypeptide may also be conjugated with another sequence or linker to identify, purify, or synthesize the polypeptide.

As used herein, the term 'homology' or 'identity' refers to the degree of similarity between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et at, Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (*i.e*., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

The O-phosphoserine sulfhydrylase variant of the present disclosure may have an increased cysteine conversion activity compared to wild-type O-phosphoserine sulfhydrylase.

For example, the O-phosphoserine sulfhydrylase variant, which is the target enzyme for comparing the increase in cysteine conversion activity, may be Msm-T-HA2(EP 2444486 B1), but is not limited thereto.

In one example, the variant having an increased cysteine conversion activity of the present disclosure may have an increased cysteine conversion activity by about 1% or more, specifically about 5% or more, about 10% or more, about 13% or more, about 15% or more, about 20% or more, about 25% or more, about 30% or more, about 35% or more, about 40% or more, about 41% or more, about 45% or more, about 46% or more, about 50% or more, about 55% or more, about 60% or more, about 65% or more, about 70% or more, about 72% or more, or about 72.5% or more (the upper limit is not particularly limited, for example, about 200% or less, about 150% or less, about 100% or less, about 50% or less, about 40% or less, about 30% or less, about 20% or less, or about 15% or less) as compared to the cysteine conversion activity of an enzyme before modification or a non-modified enzyme, but is not limited thereto, as long as it has an increased (+) value compared to the cysteine conversion activity of an enzyme before modification or a non-modified enzyme. In another example, the variant having an increased cysteine conversion activity of the present disclosure may have an increased cysteine conversion activity by about 1.01 times or more, about 1.05 times or more, about 1.1 times or more, about 1.13 times or more, about 1.15 times or more, about 1.2 times or more, about 1.25 times or more, about 1.3 times or more, about 1.35 times or more, about 1.4 times or more, about 1.41 times or more, about 1.45 times or more, about 1.46 times or more, about 1.5 times or more, about 1.55 times or more, about 1.6 times or more, about 1.65 times or more, about 1.7 times or more, about 1.72 times or more, or about 1.725 times or more (the upper limit is not particularly limited, for example, about 10 times or less, about 5 times or less, about 3 times or less, or about 2 times or less) as compared to the cysteine conversion activity of an enzyme before modification or a non-modified enzyme, but is not limited thereto.

The cysteine conversion ability may be evaluated by measuring the amount of cysteine produced through the cysteine conversion reaction. The evaluation can measure the cysteine production amount using suitable methods known in the art. For example, HPLC (High Performance Liquid Chromatography), GC (Gas Chromatography), GC/MS (Gas Chromatography-Mass Spectrometry), LC/MS (Liquid chromatography-mass spectrometry), GPC (Gel Permeation Chromatography), or a combination thereof may be used, and the cysteine production amount may be measured using suitable methods known in the art

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48: 443-453) and Needleman program of the EMBOSS package (EMBOSS: The European Molecular Biology Open Software Suite, Rice et al., 2000, Trends Genet. 16: 276-277), *etc.,* but is not limited thereto, and sequence alignment programs, such as pairwise sequence comparison algorithms, *etc.,* known in the art may be appropriately used.

Another aspect of the present disclosure provides a polynucleotide encoding the O-phosphoserine sulfhydrylase variant of the present disclosure.

The O-phosphoserine sulfhydrylase protein of the present disclosure may be encoded by a *cysM* gene.

In one example, the *cysM* gene may be a polynucleotide encoding WP_003896302.1 derived from a microorganism of the genus *Mycobacterium,* but is not limited thereto. In another example, the *cysM* gene may be a *cysM* gene derived from a microorganism of the genus *Mycobacterium,* but is not limited thereto, and it is apparent that it includes genes of various origins encoding proteins with O-phosphoserine sulfhydrylase activity.

As used herein, the term "polynucleotide", which is a polymer of nucleotides composed of nucleotide monomers connected in a lengthy chain by a covalently bond, is a DNA or RNA strand having at least a certain length. More specifically, it may mean a polynucleotide fragment encoding the O-phosphoserine sulfhydrylase variant.

The polynucleotide encoding the O-phosphoserine sulfhydrylase variant of the present disclosure may include a nucleotide sequence encoding the O-phosphoserine sulfhydrylase variant, in which 5 amino acid residues from the C-terminus in the amino acid sequence of SEQ ID NO: 1 are deleted, and the amino acid corresponding to the position 77 from the N-terminus in the amino acid sequence of SEQ ID NO: 1 is substituted with another amino acid. In one example, the polynucleotide of the present disclosure may include a nucleotide sequence encoding the amino acid sequence represented by SEQ ID NO: 3. In a more specific embodiment of the present disclosure, the polynucleotide of the present disclosure may have or include a nucleotide sequence of SEQ ID NO: 4. Additionally, the polynucleotide of the present disclosure may consist of or essentially consist of the nucleotide sequence of SEQ ID NO: 4.

The polynucleotide of the present disclosure may undergo various modifications in the coding region within the scope that does not change the amino acid sequence of the O-phosphoserine sulfhydrylase variant of the present disclosure, due to codon degeneracy or in consideration of the codons preferred in an organism in which the O-phosphoserine sulfhydrylase variant of the present disclosure is to be expressed. Specifically, the polynucleotide of the present disclosure may have or include a nucleotide sequence having a homology or identity of 70% or more, 75% or more, 80% or more, 85% or more, 90% or more, 95% or more, 96% or more, 97% or more, 98% or more, and less than 100% to the nucleotide sequence of SEQ ID NO: 4, or may consist of or essentially consist of a nucleotide sequence having a homology or identity of 70% or more, 75% or more, 80% or more, 85% or more, 90% or more, 95% or more, 96% or more, 97% or more, 98% or more, and less than 100% to the nucleotide sequence of SEQ ID NO: 4, but is not limited thereto. In particular, in the sequence having the above homology or identity, the codon encoding the amino acid corresponding to the 93^{rd} position of SEQ ID NO: 1 may be one of the codons encoding an amino acid other than proline, for example, alanine.

Additionally, the polynucleotide encoding the O-phosphoserine sulfhydrylase variant of the present disclosure may include a nucleotide sequence encoding the O-phosphoserine sulfhydrylase variant, which is a polypeptide in which 5 amino acid residues from the C-terminus in the amino acid sequence of SEQ ID NO: 1 are deleted, and the amino acid corresponding to the position 77 from the N-terminus in the amino acid sequence of SEQ ID NO: 1 is substituted with an amino acid other than the amino acid, and additionally, the amino acid corresponding to the position selected from the group consisting of 7, 55, 218, and a combination thereof from the N-terminus in the amino acid sequence of SEQ ID NO: 1 is substituted with an amino acid other than the amino acid before substitution. In one example, the polynucleotide of the present disclosure may include a nucleotide sequence encoding any one or more amino acid sequences selected from the group consisting of SEQ ID NOS: 5, 7, and 9. In a more specific embodiment of the present disclosure, the polynucleotide of the present disclosure may have or include a nucleotide sequence of SEQ ID NO: 6, SEQ ID NO: 8, or SEQ ID NO: 10. Additionally, the polynucleotide of the present disclosure may consist of or essentially consist of the nucleotide sequence of SEQ ID NO: 6, SEQ ID NO: 8, or SEQ ID NO: 10.

Specifically, the polynucleotide of the present disclosure may have or include a nucleotide sequence having a homology or identity of 70% or more, 75% or more, 80% or more, 85% or more, 90% or more, 95% or more, 96% or more, 97% or more, 98% or more, and less than 100% to the nucleotide sequence of SEQ ID NO: 6, SEQ ID NO: 8, or SEQ ID NO: 10, or may consist of or essentially consist of a nucleotide sequence having a homology or identity of 70% or more, 75% or more, 80% or more, 85% or more, 90% or more, 95% or more, 96% or more, 97% or more, 98% or more, and less than 100% to the nucleotide sequence of SEQ ID NO: 6, SEQ ID NO: 8, or SEQ ID NO: 10, but is not limited thereto. In particular, in the sequence having the above homology or identity, the codon encoding the amino acid corresponding to the 7^{th} position of SEQ ID NO: 1 may be one of the codons encoding an amino acid other than leucine, for example, proline; the codon encoding the amino acid corresponding to the 55^{th} position of SEQ ID NO: 1 may be one of the codons encoding an amino acid other than alanine, for example, valine; and the codon encoding the amino acid corresponding to the 218^{th} position of SEQ ID NO: 1 may be one of the codons encoding an amino acid other than alanine, for example, glycine.

Additionally, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions without limitation. The "stringent conditions" refer to conditions which allow the specific hybridization between polynucleotides. Such conditions are disclosed in detail in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity, i.e., polynucleotides having 70% or more, 75% or more, 80% or more, 85% or more, 90% or more, 95% or more, 96% or more, 97% or more, 98% or more, or 99% or more hybridize with each other, while polynucleotides having a homology or identity lower than the above homology or identity do not hybridize with each other; or may include ordinary washing conditions of Southern hybridization, i.e., washing once, specifically twice or three times, at a salt concentration and a temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing polynucleotides depends on the length and degree of complementarity of the polynucleotides, and these variables are well known in the art (see Sambrook et al., supra).

Still another aspect of the present disclosure provides a vector containing the polynucleotide of the present disclosure.

The vector may be an expression vector for expressing the polynucleotide in a host cell, but is not limited thereto.

The vector of the present disclosure may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptll, pGEM, pTZ, pCL and pET, *etc.* may be used. Specifically, pUC, pDZ, pDC, pDCM2, pACYC177, pACYC184, pCL, pECCG117, pUC19, pBR322, pMW118, pCC1BAC vector, *etc.* may be used.

In one example, a polynucleotide encoding a target protein may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or a microorganism so that the polypeptide encoded by the polynucleotide can be expressed in the host cell. As long as the transformed polynucleotide can be expressed in the host cell, it does not matter whether the transformed polynucleotide is integrated into the chromosome of the microorganism and located therein or located extrachromosomally, and both cases can be included. Additionally, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct comprising all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target O-phosphoserine sulfhydrylase variant of the present disclosure.

Yet another aspect of the present disclosure provides a microorganism comprising: the O-phosphoserine sulfhydrylase variant of the present disclosure; or a polynucleotide encoding the same.

The strain of the present disclosure may include the O-phosphoserine sulfhydrylase variant of the present disclosure, a polynucleotide encoding the polypeptide, or a vector containing the polynucleotide of the present disclosure.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism comprising genetic modification to produce a desired polypeptide, protein or product.

The strain of the present disclosure may be a microorganism naturally having a cysteine-producing ability; or a microorganism in which a cysteine-producing ability has been introduced into a strain not having a cysteine-producing ability. In one example, it may be a microorganism in which the O-phosphoserine sulfhydrylase variant or a polynucleotide encoding the same has been introduced, and thus having an increased cysteine-producing ability, but is not limited thereto.

The strain of the present disclosure may be a microorganism having an increased cysteine-producing ability as compared to a parent strain not comprising the variant of the present disclosure or a wild-type strain of the genus *Mycobacterium.* The microorganism may be one in which the O-phosphoserine sulfhydrylase variant of the present disclosure having an increased cysteine conversion activity as compared to wild-type O-phosphoserine sulfhydrylase or a polynucleotide encoding the same has been introduced, and thus having an improved cysteine-producing ability. Specifically, the strain of the present disclosure may have an increased cysteine-producing ability as compared to a microorganism of the genus *Mycobacterium* comprising the wild-type O-phosphoserine sulfhydrylase having the amino acid sequence of SEQ ID NO: 1 or a polynucleotide encoding the same.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. Additionally, as used herein, the term "O-phosphoserine sulfhydrylase non-modified microorganism" may refer to a strain into which the O-phosphoserine sulfhydrylase variant disclosed herein is not introduced, or a strain before the introduction thereof. The O-phosphoserine sulfhydrylase non-modified microorganism of the present disclosure does not exclude strains containing modifications of other proteins or genes in addition to the modifications of the O-phosphoserine sulfhydrylase or a polynucleotide encoding the same.

As used herein, the "non-modified microorganism" may be used interchangeably with "strain before modification", "microorganism before modification", "non-mutant strain", "non-modified strain", "non-mutant microorganism", or "reference microorganism".

The microorganism of the present disclosure may be a microorganism comprising the O-phosphoserine sulfhydrylase variant or a polynucleotide encoding the same; or a microorganism (*e.g*., a recombinant microorganism) that has been genetically modified to include the O-phosphoserine sulfhydrylase variant or a polynucleotide encoding the same, but is not limited thereto. The "endogenous activity" refers to the activity of a particular polypeptide originally possessed by a parent strain before transformation, or a wild-type or a non-modified microorganism when a trait is altered by genetic variation due to a natural or artificial factor. The endogenous activity may be interchangeably used with "activity before modification.

The microorganism of the present disclosure is not limited by its type as long as it can produce cysteine, and may be any prokaryotic or eukaryotic microorganism, and specifically, a prokaryotic microorganism. In one example, it may include microbial strains belonging to the genus *Escherichia*, the genus *Erwinia*, the genus *Serratia*, the genus *Providencia*, the genus Corynebacterium, and the genus *Brevibacterium*, and specifically, a microorganism belonging to the genus *Escherichia*, and more specifically, *Escherichia coli*, but is not limited thereto.

In another example of the present disclosure, the recombinant microorganism of the present disclosure may be a microorganism having an enhanced cysteine-producing ability, due to enhancement of activity of the part of proteins involved in the cysteine biosynthesis pathway or weakening of activity of the part of proteins involved in the cysteine degradation pathway.

As used herein, the term "enhancement" of a polypeptide activity means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification.

The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide may be confirmed by the increase in the level of activity of the polypeptide, expression level, or the amount of product excreted from the polypeptide.

The enhancement of the activity of the polypeptide may be applied by various methods well known in the art, and the method is not limited as long as it can enhance the activity of the target polypeptide compared to that of a microorganism before modification. Specifically, genetic engineering and/or protein engineering well known to those skilled in the art, which is a common method of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the enhancement of the activity of the polypeptide of the present disclosure may be achieved by:
1) increasing the intracellular copy number of a polynucleotide encoding the polypeptide;
2) replacing the expression regulatory region of a gene encoding the polypeptide on the chromosome with a sequence having a stronger activity
3) modifying the nucleotide sequence encoding the initiation codon or 5'-UTR of a gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same;
9) regulating the cellular localization of the protein (polypeptide); or
10) a combination of two or more selected from above 1 to 9), but is not particularly limited thereto.

### More specifically,

The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide (protein) operably linked to a suitable regulatory sequence into the chromosome of a host cell (microorganism). The introduction into the chromosome may be performed by introducing a vector, which is able to insert the polynucleotide into the chromosome of a host cell (microorganism), into the host cell (microorganism), but is not limited thereto. The vector is as described above. The regulatory sequence may be native (derived from the same origin) or foreign (derived from different genes) to the coding polynucleotide sequences, or may be a mutant sequence thereof or other artificial sequences, and may induce the expression of the polynucleotide in the host cell (microorganism). In one embodiment, the regulatory sequence of the *cysM* gene encoding the O-phosphoserine sulfhydrylase (OPSS) derived from *Mycobacterium smegmatis* of the present disclosure may be a PcysK promoter, but is not limited thereto.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting protein (polypeptide), but is not limited thereto.

In one embodiment, the enhancement of the activity of the O-phosphoserine sulfhydrylase may be achieved by modifying the expression regulatory region of a gene encoding the polypeptide on the chromosome of 2), by modifying the amino acid sequence or polynucleotide sequence of 4) and 5), or a combination thereof.

In any one of the above-described embodiments, the enhancement of the activity of the O-phosphoserine sulfhydrylase may include a gene expression regulatory sequence with enhanced activity in the upstream of the *cysM* gene encoding the same. For example, the gene expression regulatory sequence may be a promoter, but is not limited thereto. In another embodiment of the above-described embodiments, the enhancement of the activity of the O-phosphoserine sulfhydrylase may be achieved by modifying the amino acid sequence of the O-phosphoserine sulfhydrylase or a polynucleotide sequence encoding the same. The modification of the amino acid sequence or the polynucleotide sequence may include all of deletion, substitution, insertion, *etc.* The modification on the sequence is as described above.

Such enhancement of the polypeptide activity may mean that the activity or concentration of the corresponding polypeptide is increased relative to the activity or concentration of the polypeptide expressed in a wild-type strain or a microbial strain before modification, or that the amount of product produced from the polypeptide is increased, but is not limited thereto.

The modification of a part or all of the polynucleotide in the microorganism of the present disclosure (a) may be achieved by homologous recombination using a vector for chromosomal insertion in the microorganism or genome editing using an engineered nuclease (*e.g*., CRISPR-Cas9), and/or (b) may be induced by light, such as ultraviolet rays and radiation, *etc*. and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

As used herein, the term "weakening" of the activity of a polypeptide (e.g., proteins specified by the name of each enzyme) is a comprehensive concept comprising both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to mutation of a polynucleotide encoding the polypeptide; a case where the overall level of intracellular polypeptide activity and/or concentration (expression level) is decreased compared to a natural strain due to the inhibition of expression of a gene of a polynucleotide encoding the polypeptide, or the inhibition of translation into the polypeptide, *etc.;* a case where the polynucleotide is not expressed at all; and/or a case where no polypeptide activity is observed even when the polynucleotide is expressed. The expression that the polypeptide activity is "inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity may be performed by any method known in the art, but the method is not limited thereto, and may be achieved by applying various methods well known in the art (*e.g*., Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide activity of the present disclosure may be achieved by:
1) deleting a part or all of a gene encoding the polypeptide;
2) modifying the expression regulatory region (expression regulatory sequence) such that the expression of a gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the gene sequence encoding the polypeptide such that the polypeptide activity is removed or weakened (*e.g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene transcript encoding the polypeptide;
6) introducing an antisense oligonucleotide (*e.g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide;
9) regulating the cellular localization of the protein (polypeptide); or
10) a combination of two or more selected from above 1 to 9), but is not particularly limited thereto.

For example,
The 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.
The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.
The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.
The 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.
The 6) method of introducing an antisense oligonucleotide (*e.g*., antisense RNA), which binds complementary to the gene transcript encoding the polypeptide, can be found in the literature [Weintraub, H. et al., Antisense-RNA as a molecular tool for genetic analysis, Reviews - Trends in Genetics, Vol. 1(1) 1986].
The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.
The 8) reverse transcription engineering (RTE), which adds a promoter, which is to be reversely transcribed, on the 3' terminus of the open reading frame (ORF) of the gene sequence encoding the polypeptide, may be achieved by forming an antisense nucleotide complementary to the gene transcript encoding the polypeptide to weaken the activity.
Further, the 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting protein (polypeptide), but is not limited thereto.

Such weakening of the polypeptide activity may mean that the activity or concentration of the corresponding polypeptide is weakened relative to the activity or concentration of a polypeptide expressed in a wild-type strain or a microbial strain before modification, or that the amount of products produced from the corresponding polypeptide is decreased, but is not limited thereto.

In the microorganism of the present disclosure, the O-phosphoserine sulfhydrylase variant, polynucleotide, and cysteine, *etc.,* are as described in other aspects above.

Even another aspect of the present disclosure provides a method for producing cysteine or a derivative thereof, comprising reacting a mixture of O-phosphoserine and a sulfide; with the O-phosphoserine sulfhydrylase variant of the present disclosure.

The method for producing cysteine or a cysteine derivative of the present disclosure may include culturing the microorganism, which includes the O-phosphoserine sulfhydrylase variant of the present disclosure, the polynucleotide of the present disclosure, or the vector of the present disclosure, in a medium, prior to carrying out the method.

As used herein, the term "cultivation" means that the microorganism is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* and amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc*.; and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc*. during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for about 10 hours to 160 hours, but is not limited thereto.

The O-phosphoserine sulfhydrylase variant produced by the cultivation of the present disclosure may remain in the cells.

The method for producing cysteine of the present disclosure may further include a step of preparing the microorganism of the present disclosure, a step of preparing a medium for culturing the microorganism, or a combination thereof (regardless of the order, in any order), for example, prior to the culturing step.

The method for producing cysteine of the present disclosure may further include a step of recovering the O-phosphoserine sulfhydrylase variant from the cultured medium (medium on which the cultivation was performed), or from the microorganism of the present disclosure.

In the recovering step, the target variant may be collected using the method of culturing the microorganism of the present disclosure, for example, using a suitable method known in the art according to a batch culture, continuous culture, or fed-batch culture method, *etc.* For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the target variant can be recovered from the microorganism using suitable methods known in the art.

Additionally, the method of the present disclosure may further include a purification step after the recovering step, which may be performed using an appropriate method known in the art. In one example, when the method of the present disclosure includes both a recovering step and a purification step, the recovering step and the purification step may be performed continuously or intermittently regardless of the order, or simultaneously, or may be integrated into one step, but the method is not limited thereto.

In the method of the present disclosure, the O-phosphoserine sulfhydrylase, polynucleotide, vector, and strain, *etc.,* are as described in other aspects above.

The sulfide may be any sulfide provided not only in a solid form generally used in the art, but also in a liquid or gas form due to the difference in pH, pressure, and solubility, and thus can be converted to a thiol (SH) group in the form of sulfide (S²⁻), thiosulfate (S₂O₃²⁻), *etc*., without limitation. Specifically, the sulfide may include Na₂S, NaSH, (NH₄)₂S, H₂S or Na₂S₂O₃, which can provide a thiol group to OPS, but is not limited thereto. In the reaction, a single thiol group is supplied to a single reactive OPS group to produce a single cysteine or a derivative thereof. Herein, a sulfide may be added in an amount of 0.1 to 3 molar equivalents, and specifically 1 to 2 molar equivalents based on the molar concentration of OPS, but is not limited thereto.

Additionally, in the present disclosure, the method for producing cysteine may further include a step of recovering cysteine produced by the reaction step above. Herein, the desired cysteine may be collected by isolating and purifying cysteine from the reaction solution using a suitable reaction known in the art.

As used herein, the term "derivative" refers to similar compounds obtained by chemically modifying a portion of any compound. The term usually refers to compounds in which a hydrogen atom or a particular atom group is substituted with another atom or atom group.

As used herein, the term "cysteine derivative" refers to compounds in which a hydrogen atom or a particular atom group in cysteine is substituted with another atom or atom group. For example, the cysteine derivatives may have a form in which the nitrogen atom of the amine group (-NH₂) or the sulfur atom of the thiol group (-SH) in cysteine has another atom or atom group attached thereto, and the examples of cysteine derivatives may include N-acetylcysteine (NAC), S-Carboxymetylcysteine (SCMC), BOC-CYS(ME)-OH,(R)-S-(2-Amino-2-carboxyethyl)-L-homocysteine, (R)-2-Amino-3-sulfopropionic acid, D-2-Amino-4-(ethylthio)butyric acid, 3-sulfino-L-alanine, Fmoc-Cys(Boc-methyl)-OH, Seleno-L-cystine, S-(2-Thiazolyl)-L-cysteine, S-(2-Thienyl)-L-cysteine, S-(4-Tolyl)-L-cysteine, *etc.,* but are not limited thereto.

As long as cysteine is produced according to the method of the present disclosure, conversion to cysteine derivatives may be easily carried out by conversion into various cysteine derivatives by a method well known in the art.

In the present disclosure, the method for producing cysteine derivatives may further include converting the cysteine produced above into a cysteine derivative.

Specifically, in the present disclosure, the method for producing cysteine derivatives may include the steps of: producing cysteine according to the method of the present disclosure described above; and converting the produced cysteine into a cysteine derivative.

The conversion of the produced cysteine into a cysteine derivative may be performed by a method well known in the art, for example, cysteine may be synthesized into N-acetylcysteine (NAC) by a reaction with an acetylation agent, or may be synthesized into S-carboxymethylcysteine (SCMC) by a reaction with a haloacetic acid in basic conditions according to methods known in the art, but the examples are not limited thereto.

These cysteine derivatives are used mainly as pharmaceutical materials for antitussive agents, cough-relieving agents, and therapeutic agents for bronchitis, bronchial asthma, laryngopharyngitis, *etc.,* but are not limited thereto.

Further another aspect of the present disclosure provides a composition for producing cysteine or a cysteine derivative, comprising: the O-phosphoserine sulfhydrylase variant of the preset disclosure; a polynucleotide encoding the same; a vector containing the polynucleotide; or a microorganism comprising the polynucleotide of the present disclosure; a medium on which the microorganism is cultured; or a combination of two or more thereof.

The composition of the present disclosure may further include any suitable excipient commonly used in compositions for producing cysteine or cysteine derivatives, and such excipients may include, for example, preservatives, wetting agents, dispersing agents, suspending agents, buffers, stabilizers, or isotonic agents, *etc.,* but are not limited thereto.

Still further another aspect of the present disclosure provides a method for producing a recombinant microorganism for producing O-phosphoserine, comprising: introducing the O-phosphoserine sulfhydrylase variant of the preset disclosure; the polynucleotide of the present disclosure; or the vector of the present disclosure into a microorganism.

Still further another aspect of the present disclosure provides the use of the O-phosphoserine sulfhydrylase variant of the preset disclosure for the production of cysteine or a cysteine derivative.

Still further another aspect of the present disclosure provides the use of a recombinant microorganism for producing O-phosphoserine, which includes, the O-phosphoserine sulfhydrylase variant of the preset disclosure; the polynucleotide of the present disclosure; or the vector of the present disclosure, for the production of cysteine or a cysteine derivative.

The O-phosphoserine sulfhydrylase variant, polynucleotide, vector, cysteine, cysteine derivatives, and microorganism of the above aspects are as described above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Selection of CysM Variants

A variant library of the *cysM* gene encoding the CysM variants was prepared by random mutagenesis using an error-prone PCR kit (Diversity PCR random mutagenesis kit, Clontech) based on Msm-T-HA2 (US 9127324 B2, SEQ ID NO: 11), which is a variant in which 5 amino acid residues are deleted from the C-terminus, and proline, the 77^{th} amino acid residue from the N-terminus, is replaced with serine, in the CysM (Msm-OPSS, SEQ ID NO: 1) derived from the wild-type *Mycobacterium smegmatis* having O-phosphoserine sulfhydrylase (OPSS) activity, which exhibits the activity of converting O-phosphoserine (OPS) to cysteine.

Specifically, random mutagenesis PCR was performed using a primer pair of SEQ ID NOS: 16 and 17 based on the nucleotide sequence (SEQ ID NO: 12) encoding Msm-T-HA2 as a template. The Diversify^{™} PCR Random Mutagenesis Kit was used, and the PCR was performed under PCR amplification conditions of denaturation at 94°C for 5 minutes, followed by 20 cycles of denaturation at 94°C for 30 seconds, annealing at 55°C for 30 seconds, and polymerization at 72°C for 1 minute, and then polymerization at 72°C for 5 minutes.

The thus-amplified mutant gene fragments were each introduced into the pUC vector containing the cysK promoter (Yanisch-Perron et al. Improved M13 phage cloning vectors and host strains: nucleotide sequences of the M13mp18 and pUC19 vectors. 1985, Gene 33 (1), 103-119) (SEQ ID NO: 15, pUC_PcysK). In order to prepare the pUC_PcysK vector, PCR was performed using a primer pair of SEQ ID NOS: 18 and 19 based on the E. *coli* ATCC27325 chromosomal DNA as a template to first obtain the cysK promoter fragments. The PCR was performed under PCR amplification conditions of denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes. The thus-amplified cysK promoter fragments were cloned with the pUC vector cleaved with the Nhel and Hindlll restriction enzymes using an in-fusion cloning kit (In-fusion cloning kit, Clontech Laboratories, Inc.) to prepare the pUC_PcysK vector. The previously amplified mutant gene fragments were cloned with the pUC_PcysK vector cleaved with the EcoRV restriction enzyme using an in-fusion cloning kit. The cloning was performed by reacting at 50°C for 60 minutes, and a pUC_PcysK-cysM gene variant plasmid library was prepared therefrom.

The primer sequences used herein are shown in Table 1 below.

**[Table 1]**

| SEQ ID NO: | Name of Sequence | Sequence (5'->3') |
|---|---|---|
| 16 | cysM-mut-F | AGTTAAGGAGATATCATGACGCGCTACGACTCCCT G |
| 17 | cysM-mut-R | AGCCTTTCGTTTTATTTATTCCAGCGCGTCCTCGGC |
| 18 | PcysK-F | TTACGCCAAGCTAGCCCAGCCTGTTTACGATGATC |
| 19 | PcysK-R | GTCGTAGCGCGTCATGATATCTCCTTAACTGTATGA |

The thus-prepared enzyme expression vector was transformed into the E. *coli* K12 strain by electroporation (Appl. Microbiol.Biotechnol. (1999) 52:541-545), and homologous recombination was introduced on the chromosome (van der Rest et al., Appl Microbiol Biotechnol 52:541-545, 1999). The strains, in which the vector was inserted into the chromosome by recombination of homologous sequences, were selected from a medium containing 25 mg/L kanamycin.

DNA was obtained from the three selected *E*. *coli* transformants using a DNA-spin plasmid DNA purification kit (Intron) according to the manufacturer's protocol, and the nucleotide sequences were analyzed using sequencing. As a result of the sequencing, it was confirmed that the CysM variants derived from the three *E*. *coli* transformants consisted of the amino acid sequence of SEQ ID NO: 5, the amino acid sequence of SEQ ID NO: 7, and the amino acid sequence of SEQ ID NO: 9, respectively. In case of the CysM variant consisting of the amino acid sequence of SEQ ID NO: 5, it was confirmed that proline, the 77^{th} amino acid residue, was substituted with alanine, and alanine, the 218^{th} amino acid residue, was substituted with glycine, in the amino acid sequence of the CysM variant derived from *Mycobacterium smegmatis,* which is the parent sequence. In case of the CysM variant consisting of the amino acid sequence of SEQ ID NO: 7, it was confirmed that alanine, the 55^{th} amino acid residue, was substituted with valine, proline, the 77^{th} amino acid residue, was substituted with alanine, and alanine, the 218^{th} amino acid residue, was substituted with glycine, in the amino acid sequence of the CysM variant derived from *Mycobacterium smegmatis,* which is the parent sequence. In case of the CysM variant consisting of the amino acid sequence of SEQ ID NO: 9, it was confirmed that leucine, the 7^{th} amino acid residue, was substituted with proline, proline, the 77^{th} amino acid residue, was substituted with alanine, and alanine, the 218^{th} amino acid residue, was substituted with glycine, in the amino acid sequence of the CysM variant derived from *Mycobacterium smegmatis,* which is the parent sequence.

### Example 2: Preparation of CysM Variant Expression Vectors and Expression Strains

### 2-1. Preparation of CysM Variant (P77A or P77S) Expression Vectors and Expression Strains

In order to prepare strains expressing the CysM variants solely containing the mutation confirmed in Example 1, CysM variant expression vectors were first prepared.

Specifically, in order to prepare the CysM(P77A) expression vector, the upstream fragments of the *cysM*(P77A) gene were obtained through PCR using a primer pair of SEQ ID NOS: 16 and 20, based on the nucleotide sequence (SEQ ID NO: 14) encoding the Msm-T (US 9127324 B2) (SEQ ID NO: 13), which is a variant in which 5 amino acid residues were deleted from the C-terminus of CysM (Msm-OPSS) derived from the wild-type *Mycobacterium smegmatis,* as a template, and the downstream fragments of the cysM(P77A) gene were obtained through PCR using a primer pair of SEQ ID NOS: 17 and 21 based on the same template. In order to prepare the CysM(P77S) (Msm-T-HA2) expression vector as a control, the *cysM*(P77S) gene fragments were obtained through PCR using a primer pair of SEQ ID NOS: 16 and 17 based on the Msm-T-HA2 as a template. The PCR was performed under PCR amplification conditions of denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes.

The thus-obtained gene fragments were each introduced into the pUC vector (pUC_PcysK) containing the cysK promoter. The vector prepared in Example 1 was used as the pUC_PcysK vector. The previously obtained *cysM*(P77A) and *cysM*(P77S) gene fragments were cloned with the pUC_PcysK vector cleaved with the EcoRV restriction enzyme using an in-fusion cloning kit. The cloning was performed by reacting at 50°C for 60 minutes.

The primer sequences used herein are shown in Table 2 below.

**[Table 2]**

| SEQ ID NO: | Name of Sequence | Sequence (5'->3') |
|---|---|---|
| 16 | cysM-mut-F | AGTTAAGGAGATATCATGACGCGCTACGACTCCCT G |
| 20 | P77A-Reverse | GTGTTGCCGCTGGTGGCTTCCAGGATCGTG |
| 21 | P77A-Forward | CACGATCCTGGAAGCCACCAGCGGCAACAC |

Based on this, pUC_PcysK-cysM(P77A) and pUC_PcysK-cysM(P77S) (Msm-T-HA2) vector were obtained, respectively, and the vectors were transformed into the *E*. *coli* K12 strain in the same manner as in Example 1 to obtain CysM variant (P77A or P77S) expression strains.

### 2-2. Preparation of CysM Variant (P77A/A218G) Expression Vectors and Expression Strains

In order to prepare strains expressing the CysM variants containing two combinations of the mutations confirmed in Example 1, CysM variant expression vectors were first prepared.

Specifically, in order to prepare the cysM(P77A/A218G) expression vector, the upstream fragments of the *cysM*(P77A/A218G) gene were obtained through PCR using a primer pair of SEQ ID NOS: 16 and 22, based on the cysM-4 (SEQ ID NO: 3) as a template, and the downstream fragments of the *cysM*(P77A/A218G) gene were obtained through PCR using a primer pair of SEQ ID NOS: 17 and 23 based on the same template. The PCR was performed under PCR amplification conditions of denaturation at 94°C for 5 minutes, followed by 30 cycles of denaturation at 94°C for 30 seconds, annealing at 55°C for 30 seconds, and polymerization at 72°C for 1 minute, and then polymerization at 72°C for 5 minutes.

The primer sequences used herein are shown in Table 3 below.

**[Table 3]**

| SEQ ID NO: | Name of Sequence | Sequence (5'->3') |
|---|---|---|
| 16 | cysM-mut-F | AGTTAAGGAGATATCATGACGCGCTACGACTCCCTG |
| 17 | cysM-mut-R | AGCCTTTCGTTTTATTTATTCCAGCGCGTCCTCGGC |
| 22 | A218G-R | GCCCTCGTCGATGTTGCGCAGgcCGTACACGCCCTCG CCGTAACG |
| 23 | A218G-F | CGTTACGGCGAGGGCGTGTACGgcCTGCGCAACATC GACGAGGGC |

The thus-obtained gene fragments were cloned with the pUC_PcysK vector of Example 1 cleaved with the EcoRV restriction enzyme using an in-fusion cloning kit. The cloning was performed by reacting at 50°C for 60 minutes.

From this, pUC_PcysK-cysM(P77A/A218G) vector was obtained, and the vector was transformed into the *E*. *coli* K12 strain in the same manner as in Example 1 to obtain CysM variant (P77A/A218G) expression strains.

### Example 3. Assessment of Cysteine Conversion Ability of CysM Variant Expression Strains

### 3-1. Assessment of Cysteine Conversion Ability of CysM Variant (P77A or P77S) Expression Strains

Expression of the enzymes was carried out according to the manual of the pET system (Novagen). Specifically, single colonies of each strain of Example 2-1 were selected from the LB plates containing kanamycin and inoculated into 5 mL of LB broth and incubated at 37°C, 200 rpm for 16 hours. Then, the strains were inoculated into the LB broth containing kanamycin and incubated at 33°C, 200 rpm for 18 hours. The enzymes were obtained by adding xylene (2% of the culture broth) to the culture broth. The enzymes obtained through the above process were each confirmed through 14% SDS-PAGE (FIG. 1), and soluble CysM(Msm-OPSS) variants were obtained.

The soluble CysM variants K12/pUC_Ppro-cysM(P77S) (Msm-T-HA2) and K12/pUC_Ppro-cysM(P77A) obtained above were used to compare and analyze the enzymatic activity of converting OPS as a substrate to cysteine. The conditions for cysteine synthesis activity assay (CysM enzyme assay) are shown in Table 4 below.

**[Table 4]**

| Stock sol'n | Final Conc. | Volume |
|---|---|---|
| 10x his-enzyme | | 25 µL |
| 0.6M OPS in 1M Tris-HCl(pH 8.5) | 300mM | 500 µL |
| 1.5M Na₂S-9H₂O in D.W | 600mM | 380 µL |
| 10 mM PLP in D.W | 0.2mM | 20 µL |
| D.W | 0.2mM | 75 µL |
| Total | | 1000 µL |

In Table 1, the remaining solutions excluding the enzymes were mixed and incubated at 37°C for 5 minutes, then 25 µL of the xylene-treated soluble CysM variants were added and reacted at 37°C for 10 minutes. After the reaction was completed, 100 µL of the reaction solution was taken and reacted with 900 µL of a mixture containing 100 mM Tris-HCl (pH 8.5) and 10 mM DTT (dithiothreitol) for 30 minutes, and then the reaction was terminated by adding 0.1M HCl. The concentration of cysteine in the reaction solution was quantified through HPLC, and the cysteine synthesis titer was assessed by comparing the cysteine conversion rate and specific activity (cysteine concentration/time/enzyme amount) during the 10-minute reaction, and the results are shown in Table 5 below.

**[Table 5]**

| Name of Strains | Specific activity (cysteine(µmole)/min/mg) |
|---|---|
| K12/pUC_PcysK-cysM(P77S) (Msm-T-HA2) | 12.6 |
| K12/pUC_PcysK-cysM(P77A) | 18.4 |

As shown in the results in Table 5 above, the *cysM*(P77A) expression strain showed an improvement in cysteine synthesis activity with a 46% increase in specific activity compared to the *cysM*(P77S) (Msm-T-HA2) expression strain, which is the control.

### 3-2. Assessment of Cysteine Conversion Ability of CysM Variant (P77A/A218G) Expression Strains

The K12/pUC_PcysK-cysM(P77A/A218G), the soluble CysM variant obtained by the method of Example 2-2, was used to compare and analyze the enzymatic activity of converting OPS as a substrate to cysteine. The experiment was performed in the same manner as Example 3-1. The concentration of cysteine in the reaction solution was quantified through HPLC, and the cysteine synthesis titer was assessed by comparing the cysteine conversion rate and specific activity (cysteine concentration/time/enzyme amount) during the 10-minute reaction, and the results are shown in Table 6 below.

**[Table 6]**

| Name of Strain | Specific activity (cysteine(µmole)/min/mg) |
|---|---|
| K12/pUC_PcysK-cysM(P77A) | 18.2 |
| K12/pUC_PcysK-cysM(P77A/A218G) | 43.5 |

As shown in the results in Table 6 above, the *cysM*(P77A/A218G) expression strain showed an improvement in cysteine synthesis activity with a 13% increase in specific activity compared to the *cysM*(P77S) expression strain, which is the control.

### 3-3. Assessment of Cysteine Conversion Ability of CysM Variant (L7P/P77A/A218G or A55V/P77A/A218G) Expression Strains

With respect to the CysM variants, K12/pUC_PcysK-cysM(P77A/A218G), K12/pUC_PcysK-cysM(L7P/P77A/A218G), and K12/pUC_PcysK-cysM(A55V/P77A/A218G), which were obtained through screening in the library prepared as a result of Example 1, the enzymatic activity of converting OPS as a substrate to cysteine was compared and analyzed. The experiment was performed in the same manner as Example 3-1. The concentration of cysteine in the reaction solution was quantified through HPLC, and the cysteine synthesis titer was assessed by comparing the cysteine conversion rate and specific activity (cysteine concentration/time/enzyme amount) during the 10-minute reaction, and the results are shown in Table 7 below.

**[Table 7]**

| Name of Strain | Specific activity (cysteine(µmole)/min/mg) |
|---|---|
| K12/pUC_PcysK-cysM(P77A/A218G) | 44.3 |
| K12/pUC_PcysK-cysM(L7P/P77A/A218G) | 62.8 |
| K12/pUC_PcysK-cysM(A55V/P77A/A218G) | 76.4 |

As shown in the results in Table 7 above, the *cysM*(L7P/P77A/A218G) expression strain and the *cysM*(A55V/P77A/A218G) expression vector showed an improvement in cysteine synthesis activity with 41% and 72.5 increase in specific activity, respectively, compared to the *cysM*(P77A/A218G) expression strain, which is the control.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. O-phosphoserine sulfhydrylase variant, in which 0 to 7 amino acid residues from the C-terminus in the amino acid sequence of SEQ ID NO: 1 are deleted, and the amino acid corresponding to the position 77 is substituted with alanine.

2. The variant of claim 1, wherein 5 amino acid residues from the C-terminus in the amino acid sequence of SEQ ID NO: 1 are deleted.

3. The variant of claim 1, having at least 80% homology or identity of with SEQ ID NO: 1.

4. The variant of claim 1, consisting of an amino acid sequence of SEQ ID NO: 3

5. The variant of claim 1, wherein the amino acid corresponding to the positions selected from the group consisting of 7, 55, 218, and a combination thereof in the amino acid sequence of SEQ ID NO: 1 is further substituted with another amino acid.

6. The variant of claim 5, wherein the amino acid corresponding to the position 7 in the amino acid sequence of SEQ ID NO: 1 is substituted with proline.

7. The variant of claim 5, wherein the amino acid corresponding to the position 55 in the amino acid sequence of SEQ ID NO: 1 is substituted with valine.

8. The variant of claim 5, wherein the amino acid corresponding to the position 218 in the amino acid sequence of SEQ ID NO: 1 is substituted with glycine.

9. The variant of claim 5, consisting of any one or more amino acid sequences selected from the group consisting of SEQ ID NOS: 5, 7 and 9.

10. A polynucleotide encoding the O-phosphoserine sulfhydrylase variant of any one of claims 1 to 9.

11. A microorganism, comprising the O-phosphoserine sulfhydrylase variant of any one of claims 1 to 9; or a polynucleotide encoding the same.

12. The microorganism of claim 11, having an increased cysteine-producing ability compared to a microorganism comprising wild-type O-phosphoserine sulfhydrylase having the amino acid sequence of SEQ ID NO: 1 or a polynucleotide encoding the same.

13. The microorganism of claim 12, wherein the microorganism belongs to the genus *Escherichia.*

14. The microorganism of claim 13, wherein the microorganism belonging to the genus *Escherichia* is *Escherichia coli.*

15. A method for producing cysteine or a derivative thereof, comprising reacting a mixture of O-phosphoserine and a sulfide; with the O-phosphoserine sulfhydrylase variant of any one or claims 1 to 9.

16. The method of claim 15, wherein the O-phosphoserine is purified O-phosphoserine or a microbial fermented liquid comprising O-phosphoserine.

17. The method of claim 15, wherein the sulfide is one or more selected from the group consisting of Na₂S, NaSH, (NH₄)₂S, H₂S, S₂O₃ and Na₂S₂O₃.

18. Use of an O-phosphoserine sulfhydrylase variant, in which 0 to 7 amino acid residues are deleted from the C-terminus in the amino acid sequence of SEQ ID NO: 1, and the amino acid corresponding to position 77 is substituted with alanine, for the production of cysteine or a derivative thereof.

19. Use of a microorganism, which comprises the O-phosphoserine sulfhydrylase variant of any one or claims 1 to 9; a polynucleotide encoding the same; or a vector comprising the polynucleotide, for the production of cysteine or a derivative thereof.
